# EUROPÄISCHE PATENTANMELDUNG

(11) **EP 2 311 449 A1**
(43) Veröffentlichungstag der Anmeldung: **20.04.2011**
(21) Anmeldenummer: 09450195.4
(22) Anmeldetag: 14.10.2009
(51) Int. Cl.: A61K 31/205, A61K 31/05, A61K 31/375, A61K 31/685, A61K 45/06, A23L 1/30

(54) **Oral verabreichbare nahrungsergänzende Zusammensetzung enthaltend Carnitin**

(71) Anmelder: NÖM Aktiengesellschaft, 2500 Baden (AT)
(72) Erfinder: Lohninger, Alfred Dr., A-3521 Gaaden (AT)
(74) Vertreter: Keschmann, Marc

(57) **Zusammenfassung**

Bei einer oral verabreichbaren Zusammensetzung zur Verbesserung der Energiegewinnung aus Fettsäuren im oxidativen Abbau enthaltend L-Carnitin sind L-Carnitin und/oder dessen wasserlösliche Salze bzw. C2- oder C3-Ester gemeinsam mit mit essentiellen Fettsäuren veresterten Phospholipiden, insbesondere in Form von Phosphatidylcholin (Lecithin) und/oder Phosphatidylglycerol, sowie Polyphenole als Antioxidantien enthalten.

SEQUENCE LISTING

<110>  NOEM AG

<120>  ORAL VERABREICHBARE NAHRUNGSERGAENZENDE ZUSAMMENSETZUNG

<130>  44313

<140>  EP 09 450 195.4
<141>  2009-10-14

<160>  12

<170>  PatentIn version 3.5

<210>  1
<211>  19
<212>  DNA
<213>  Artificial Sequence

<220>
<223>  Synthetic primer

<400>  1
gtcccggctg tcaaagaca                                                    19


<210>  2
<211>  21
<212>  DNA
<213>  Artificial Sequence

<220>
<223>  Synthetic primer

<400>  2
ccgacagcaa aatcttgagc a                                                 21


<210>  3
<211>  20
<212>  DNA
<213>  Artificial Sequence

<220>
<223>  Synthetic primer

<400>  3
caggcgagaa cacgatcttc                                                   20


<210>  4
<211>  19
<212>  DNA
<213>  Artificial Sequence

<220>
<223>  Synthetic primer

<400>  4
gcggatgtgg tttccaaag                                                    19


<210>  5
<211>  15
<212>  DNA
<213>  Artificial Sequence

<220>
<223>  Synthetic primer

<400>  5
gaagcccttc tcctt                                                        15


<210>  6
<211>  19
<212>  DNA
<213>  Artificial Sequence

<220>
<223>  Synthetic primer

<400>  6
ctcccctaca cctcctgag                                                    19


<210>  7
<211>  20
<212>  DNA
<213>  Artificial Sequence

<220>
<223>  Synthetic primer

<400>  7
cagggctgct tttaactctg                                                   20


<210>  8
<211>  22
<212>  DNA
<213>  Artificial Sequence

<220>
<223>  Synthetic primer

<400>  8
catgacgaac atgggggcat cc                                                22

<210>  9
<211>  25
<212>  DNA
<213>  Artificial Sequence

<220>
<223>  Synthetic primer

<400>  9
ccgcatcgac cactacctgg gcaag                                              25


<210>  10
<211>  26
<212>  DNA
<213>  Artificial Sequence

<220>
<223>  Synthetic primer

<400>  10
gttccccacg tactggccca ggacca                                             26


<210>  11
<211>  18
<212>  DNA
<213>  Artificial Sequence

<220>
<223>  Synthetic primer

<400>  11
tgccatccta aaagccac                                                      18


<210>  12
<211>  21
<212>  DNA
<213>  Artificial Sequence

<220>
<223>  Synthetic primer

<400>  12
tcaactggtc tcaagtcagt g                                                  21

## Beschreibung

Die Erfindung betrifft eine oral verabreichbare nahrungsergänzende Zusammensetzung zur Verbesserung der Energiegewinnung aus Fettsäuren im oxidativen Abbau enthaltend L-Carnitin.

Aus der EP 1 014 965 B1 ist eine oral verabreichbare trockene Einheitsdosierung bekannt geworden, welche Carnitin und ein mitochondrial aktives Antioxidans, welches physiologisch eine metabolisch reaktive Thiolgruppe umfasst, enthält. Derartige Darreichungsformen bzw. Einheitsdosierungen erlauben es, den Metabolismus zu steigern und oxidativen Stress zu lindern. Prinzipiell sind derartige Produkte als Nahrungsergänzung geeignet und verlangsamen den biologisch bedingten Alterungsprozess. Gleichzeitig werden natürliche Abwehrkräfte gestärkt, die Merk-, Lern- und Leistungsfähigkeit des Gehirns verbessert und der oxidative Stoffwechsel verjüngt. Unter Verjüngung des oxidativen Stoffwechsels versteht man hierbei, dass vermehrt Fettsäuren zur Energiegewinnung im oxidativen Stoffwechsel verwertet werden, was den Herz- und Skelettmuskeln eine erhöhte Energieproduktion ermöglicht.

Unausgewogene Ernährung, die Verringerung des Nährstoffgehaltes bei industriell gefertigten Nahrungsmitteln, hohe Umwelt- und Stressbelastung, hoher Kaffee-, Tabak-, Alkohol- und Arzneimittelkonsum, biologisch bedingte Alterungsprozesse und vieles andere mehr belasten den Organismus und beeinträchtigen das Wohlbefinden und die Gesundheit. Da die meisten dieser Belastungen mit einer vermehrten Bildung von Radikalen verbunden sind, wurde bereits vorgeschlagen zur Verlangsamung des biologisch bedingten Alterungsprozesses Radikalfänger bzw. Antioxidantien zuzusetzen. Wenn der oxidative Stoffwechsel stimuliert wird, ist es in der Regel nicht vermeidbar, dass aus rund 2% des umgesetzten Sauerstoffs Sauerstoffradikale entstehen. Der Zusatz von Antioxidantien erlaubt es somit, diese zusätzlich entstehenden Sauerstoffradikale abzufangen.

Für die Wirksamkeit der den oxidativen Fettsäureabbau stimulierenden Substanzen, und insbesondere von Carnitin, ist aber vor allem eine entsprechende intrazelluläre Konzentration notwendig. Wenn der Transport von Carnitin in die Zelle durch die Zellmembran nicht hinreichend gelingt, ist auch der Zusatz von Carnitin in nahrungsergänzenden Zusammensetzungen weitestgehend wirkungslos. Carnitin kann aber auch im Darm bakteriell abgebaut werden bevor es wirksam werden kann.

Für die Carnitinaufnahme wurden Na⁺ abhängige und Na⁺ unabhängige Transportsysteme aufgefunden, die sich auch in ihrer Affinität unterscheiden. Die intrazelluläre Konzentration des Carnitins wird hiebei durch verschiedene Membrantransporter kontrolliert, wobei der humane Kationentransporter OCTN2 physiologisch besonders wichtig ist. Die Expression von OCTN2 nimmt mit zunehmendem Alter und bei hohen Konzentrationen an Sauerstoffradikalen ab.

In der EP 1 586 317 wurde eine oral verabreichbare Zusammensetzung zur Verbesserung der Energiegewinnung aus Fettsäuren im oxidativen Abbau enthaltend L-Carnitin geoffenbart, bei welcher L-Carnitin zusammen mit Methylxanthinen sowie veresterten essentiellen Fettsäuren in Form von essentiellen Phospholipiden eingesetzt sind. Es ist weiters bekannt, Koffein und Arginin zusammen mit Isoflavonen aus Soja und L-Carnitin in einer oral verabreichbaren Formulierung darzureichen, um eine positive Stimulierung des Fettmetabolismus und folglich eine positive Beeinflussung von Fettleibigkeitssymptomen zu erreichen.

Ausgehend von diesem Stand der Technik zielt die Erfindung nun darauf ab, die positiven Effekte auf den Fettstoffwechsel weiter zu verbessern.

Zur Lösung dieser Aufgabe besteht die erfindungsgemäße oral verabreichbare Zusammensetzung enthaltend L-Carnitin im Wesentliche darin, dass L-Carnitin und/oder dessen wasserlösliche Salze bzw. C₂- oder C₃-Ester gemeinsam mit mit essentiellen Fettsäuren veresterten Phospholipiden, insbesondere in Form von Phosphatidylcholin (Lecithin) und/oder Phosphatidylglycerol, sowie Polyphenole als Antioxidantien enthalten sind. Da Polyphenole und essentielle Phospholipide gemeinsam mit L-Carnitin eingesetzt werden, wird die Funktion der Mitochondrien verbessert, sodass Kennwerte von Mitochondrien, wie beispielsweise das Membranpotential oder der Cardiolipingehalt der inneren Mitochondrienmembran, wieder auf Werte ansteigen, welche mit jenen von jugendlichen Organismen vergleichbar sind. Daraus resultiert eine Verjüngung des Stoffwechsels, beispielsweise durch eine Verbesserung der Funktion der Atmungskette. Insbesondere konnte in tierexperimentellen Studien und Untersuchungen an freiwilligen Probanden gezeigt werden, dass die regelmäßige Zufuhr oder Einnahme derartiger oral verabreichbarer Zusammensetzungen die Sauerstoffaufnahme durch wichtige Gewebe, wie Gehirn, Niere und Leber, vermehrt und gleichzeitig die Spiegel an Oxidationsprodukten von Lipiden nach Lipidperoxidation, z.B. Malondialdehyd, vermindert. Von wesentlicher Bedeutung ist hiebei der Umstand, dass die intrazelluläre Konzentration von Carnitin ein entscheidender Kontrollfaktor der Transkription von zahlreichen Genen des oxidativen Stoffwechsels ist. Der mRNA-Gehalt dieser metabolischen Enzyme ist bei niedrigem Carnitinspiegel stark vermindert und kann durch Carnitinsubstitution erhöht werden. Die erhöhte mRNA-Expression von Schlüsselenzymen der Fettsäurenoxidation, und insbesondere der Carnitin-Palmitoyltransferase 1 (CPT1A) und besonders der muskelständigen Isoform (CPT1B) sowie der Carnitin-Acetyltransferase, führt zu der gewünschten Verbesserung des oxidativen Fettsäureabbaues. Die Carnitin-Acetyltransferase, welche besonders deutlich stimuliert wird, fungiert hierbei als Bindeglied zum Kohlenhydratstoffwechsel, da dieses Enzym Acetylreste von Acetyl-CoA auf Carnitin überträgt, wodurch die Konzentration an freiem Coenzym A in den Mitochondrien erhöht wird und Pyruvat wieder verstärkt verstoffwechselt und der Citratcyclus stimuliert werden kann. Besonders hervorzuheben ist, dass auch der turnover von sauren Gruppen in den Mitochondrien erhöht wird, welche sauren Gruppen als Carnitinester über das CAR-Carriersystem aus den Mitochondrien und letztlich den Zellen ausgeschleust werden können, was allgemein als Entgiftungsfunktion beschrieben ist. Auch für diesen metabolischen Schritt ist die Aktivität der Carnitin-Acetyltransferase ausschlaggebend, da die angereicherten sauren metabolischen Zwischenprodukte zunächst als CoA-Ester vorliegen und erst zu Carnitinderivaten umgeestert werden müssen. Durch die beschriebene Entgiftungsfunktion können die Spiegel an freiem Carnitin wieder sinken, obwohl die Stimulierung des oxidativen Stoffwechsels vorliegt. Dieser Vorgang ist durch eine vermehrte Ausscheidung von Carnitinestern im Urin nachweisbar. Gleichzeitig steigen der Cardiolipingehalt im Gewebe auf das Niveau jüngerer Gewebe und die intrazellulären Spiegel an, wobei Werte erzielt werden, welche mit alleiniger Carnitinzufuhr nicht erreicht werden können.

Als besonders bevorzugt gilt in diesem Zusammenhang, dass Resveratrol als Polyphenol eingesetzt ist. Resveratrol ist hierbei besonders wirksam, wobei entsprechend einer weiteren bevorzugten Ausführungsform Traubenkernextrakt enthalten ist. Traubenkernextrakt ist günstig verfügbar und weist hohe Konzentrationen der gewünschten Polyphenole auf, wobei das besonders wirksame Resveratrol, ein Polyphenol, welches insbesondere in Traubenschalen vorkommt, zugesetzt werden kann.

Gemäß einer weiteren bevorzugten Ausführungsform ist vorgesehen, dass zusätzlich Isoflavonoide als Antioxidantien enthalten sind, was die positive Wirkung der erfindungsgemäßen Zusammensetzung auf die Funktion der Mitochondrien weiter verstärkt.

Die Zugabe von essentiellen Phospholipiden dient gleichfalls der Verbesserung des Transports durch die Zellmembran, wobei hier bevorzugt als mit essentiellen Fettsäuren veresterte Phospholipide Phosphatidylcholin (Lecithin) oder Phosphatidylglycerol, insbesondere jene Spezies die mit Linol-, Linolensäure, Eicosapentaensäure und/oder Docosahexaensäure verestert sind, eingesetzt sind. Linolsäure kann hierbei zu Arachidonsäure und Linolensäure zu Eicosapentaensäure und Docosahexaensäure umgewandelt werden. Insbesondere die Produkte aus der Reihe der ω-3-Fettsäuren haben positive physiologische Effekte und insbesondere positive kardiovaskuläre Eigenschaften.

Zusätzlich zu den erfindungsgemäß vorgeschlagenen Maßnahmen zur Erhöhung der intrazellulären Carnitinkonzentration bzw. des Carnitinester-turnover kann aber auch, wie an sich bekannt, noch dem Umstand Rechnung getragen werden, dass ein stimulierter Fettsäureabbau gleichzeitig zu einer Zunahme von Radikalen führt. Es können daher zusätzlich noch bekannte Antioxidantien, und insbesondere bevorzugt Antioxidantien aus der Gruppe der Flavonoide, Isoflavonoide, insbesondere Genistein und/oder Daidzein, Catechine, β-Carotinoide, Vitamine C und Vitamine E, eingesetzt werden. In an sich bekannter Weise kann die Zusammensetzung auch Zinksalze oder -oxide enthalten.

Die erfindungsgemäßen oral verabreichbaren Zusammensetzungen können unmittelbar Nahrungsmitteln zugesetzt werden, wobei auf die maximal wünschenswerten Tagesdosen Rücksicht genommen werden muss. Bei einer Einheitsdosis sind beispielsweise Carnitinmengen von 100 mg bis 4000 mg gesundheitlich unbedenklich und wünschenswert, wobei Polyphenole in Mengen von 100 mg bis 200 mg und Phospholipide in Mengen bis zu 3000 mg zum Einsatz gelangen können. Bioflavonoide können auch als Extrakt der roten Traubenschalen eingesetzt werden, wobei die in Nahrungsergänzungsmitteln bereits weitestgehend üblichen Zusätze von Vitaminen A, E und C in den üblichen Grenzen gehalten werden.

Die Erfindung wird nachfolgend anhand eines Ausführungsbeispiels näher erläutert. In einer Studie wurde der Effekt der Gabe von L-Carnitin gemeinsam mit veresterten essentiellen Fettsäuren in Form von Phospholipiden sowie mit Polyphenolen (EPPC) auf das Plasmalipidniveau und die Expression von Enzymen, welche in die Regulation der Fettsäureoxidation in peripheren einkernigen Blutzellen (PMNC) bei Personen mit Hyperlipidämie eingreifen, untersucht. Die Gabe von EPPC führte zu einer signifikanten Verringerung der freien Fettsäuren und zu einer merklichen Verringerung der Triazyl-Glyzerin-Niveaus.

In der Studie wurden 45 Teilnehmer mit Hyperlipidämie untersucht, wobei keiner der Teilnehmer kardiovaskuläre Krankheiten, unbehandelten Bluthochdruck, Diabetes oder Nieren- und Leberkrankheiten hatte und keiner einer Medikation unterlag, welche die Serumlipide beeinflusst. Während der Studie änderten die Teilnehmer ihre Lebens- und Ernährungsgewohnheiten nicht. Den Teilnehmern wurde ein probiotischer Joghurtdrink mit niedrigem Fettgehalt enthaltend L-Carnitin (1 g), Traubenkernextrakt (81 mg), Polyphenole, Phospholipide verestert mit w-6-Fettsäuren und w-3-Fettsäuren (354 mg), Biotin (0,15 mg), Vitamin C (60 mg) und Vitamin E (10 mg) zweimal pro Tag verabreicht. Einer Kontrollgruppe wurde ein probiotischer Joghurtdrink mit niedrigem Fettgehalt enthaltend Vitamin A (0,15 mg), Vitamin C (60 mg) und Vitamin E (10 mg) zweimal täglich verabreicht.

Bei beiden Gruppen wurde nach mindestens 12 Stunden Nüchternheit venöses Blut gewonnen und die PMNC wurden mittels einer Dichtegradientenzentrifugation unter Verwendung von FICOLL-Separationsmedium angereichert. Aus diesen Zellen wurde die RNA isoliert, cDNA hergestellt und eine Realtime-PCR nach bekannten Methoden durchgeführt. Um die verschiedenen Enzyme mittels PCR zu identifizieren und zu quantifizieren, wurden folgende Primer verwendet:
**CPT1A*** (117bp): forward: 5'-GTCCCGGCTGTCAAAGACA-3' (SEQ ID NO: 1); reverse: 5'-CCGACAGCAAAATCTTGAGCA-3' (SEQ ID NO: 2);
**CPT1B*** (85bp) : forward 5'-CAGGCGAGAACACGATCTTC-3' (SEQ ID NO : 3); reverse: 5'-GCGGATGTGGTTTCCAAAG-3' (SEQ ID NO: 4);
**CRAT*:** forward: 5'-GAAGCCCTTCTCCTT-3'(SEQ ID NO: 5); reverse: 5'-CTCCCCTACACCTCCTGAG-3'(SEQ ID NO: 6);
**GAPDH*:** forward: 5'-CAGGGCTGCTTTTAACTCTG-3' (SEQ ID NO: 7); **reverse:** 5'-CATGACGAACATGGGGGCATCC-3' (SEQ ID NO: 8);
**G6PD*;** forward: 5'-CCGCATCGACCACTACCTGGGCAAG-3' (SEQ ID NO: 9); reverse: 5'-GTTCCCCACGTACTGGCCCAGGACCA-3' (SEQ ID NO: 10);
β-**Actin**: forward: 5'-TGCCATCCTAAAAGCCAC-3'(SEQ ID NO: 11); reverse: 5'-TCAACTGGTCTCAAGTCAGTG-3 (SEQ ID NO: 12)
   * CPT1A: Carnitin-Palmitoyl-Transferase
   CPT1B: Carnitin-Palmitoyl-Transferase (muskelständige Isoform)
   CRAT: Carnitin-Acetyl-Transferase
   GAPDH: Glycerinaldehyd-3-phosphat-Dehydrogenase
   G6PD: Glucose-6-phosphat-Dehydrogenase

Freie Fettsäuren, freies Cholesterin, Cholesterinester und Triazylglycerin wurden direkt mittels Kapillargaschromatographie bestimmt.

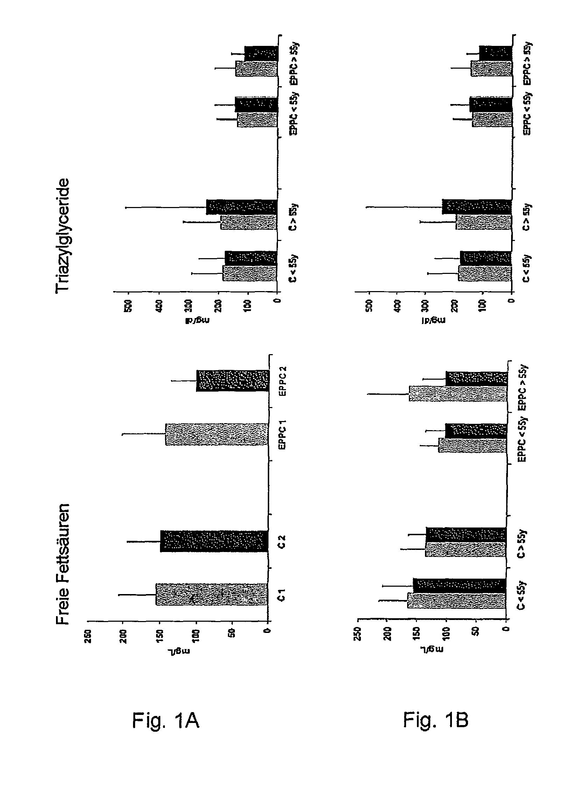

Durch die Gabe von EPPC konnte eine signifikante Verringerung der Plasmakonzentrationen der freien Fettsäuren und eine markante Reduktion der Triglycerid-Niveaus beobachtet werden (Fig. 1A). Dieser Effekt war bei Teilnehmern, welche älter als 55 Jahre waren, deutlicher ausgeprägt, als bei jüngeren Teilnehmern (Fig. 1B).

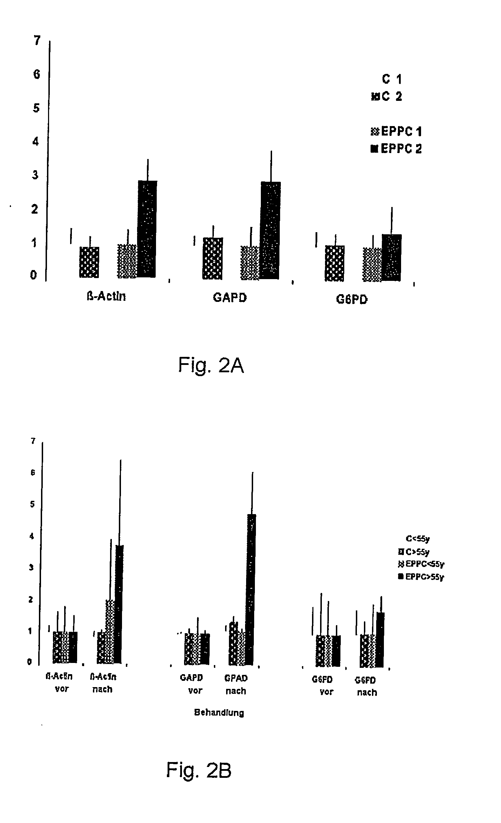

Im Gegensatz zur Kontrollgruppe (C1, C2) führte die Substitution der EPPC-Kombination zu einer beinahe dreifachen Zunahme der b-actin-mRNA und GAPD-mRNA und einer 50%-Zunahme der G6PD-mRNA-Expression (Fig. 2A). Diese Zunahmen waren wiederum bei den über 55-jährigen stärker ausgeprägt (Fig. 2B).

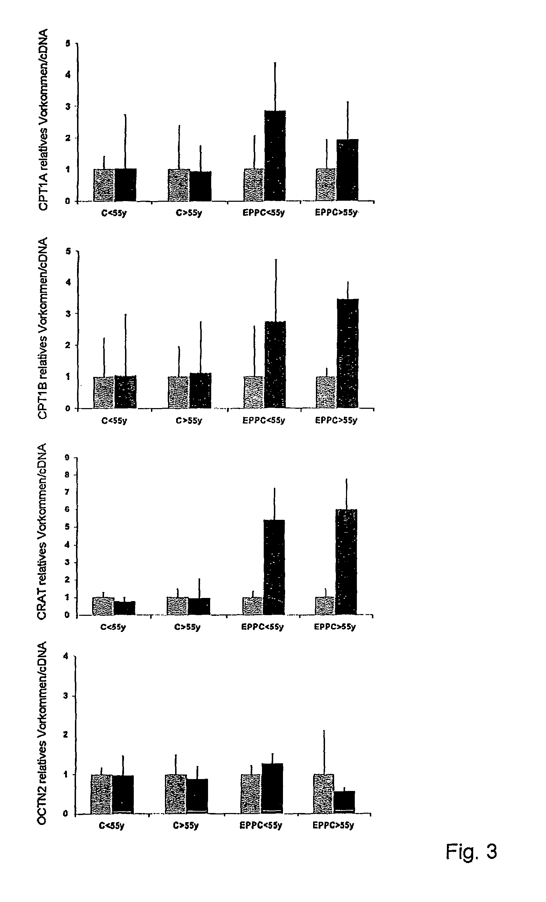

Die relativen Mengen an mRNA von CPT1A, CPT1B und CRAT waren nach EPPC-Substitution erhöht (2,5-fach, 3-fach und bzw. 6-fach). Interessanter Weise war dieser Effekt stärker bei den unter 55-jährigen für CPT1A und bei den über 55-jährigen für CPT1B. Für CRAT wurde kein solcher Effekt gefunden (Fig. 3).

In der Studie konnte gezeigt werden, dass die Gabe von EPPC in Getränken auf Joghurtbasis die Konzentration an freien Fettsäuren und die Konzentration von Triglyceriden in Plasma senken konnte, was aller Wahrscheinlichkeit nach auf die Stimulation der Fettsäureoxidation zurückzuführen ist. Die Gabe von L-Carnitin führte zu einer signifikanten Zunahme der CO₂-Exhalation, was eine Zunahme der Fettsäureoxidation in gesunden und leicht übergewichtigen Patienten ohne Carnitindefizienz anzeigt. Darüber hinaus wurde bei allen behandelten Teilnehmern eine 6-fache Zunahme der relativen CRAT-mRNA-Expression in PMNC gefunden. In der mitochondrialen Matrix katalysiert dieses Enzym ein reversibles Gleichgewicht zwischen CoA mit kurzkettigen Acylresten und CoASH und kurzkettigem A-cylcarnitin und freiem Carnitin, sodass der acylierte Anteil sowohl für CoA als auch für L-Carnitin der gleiche ist. Acylcarnitine (Acetatderivate und nicht-metabolisierte, kurzkettige Acylderivate) können in der Folge in den viel größeren cytosolischen Pool über CACT exportiert werden und über die Zellen weiter ins Blut und letztlich über die Nieren ausgeschieden werden.

## Patentansprüche

1. Oral verabreichbare nahrungsergänzende Zusammensetzung zur Verbesserung der Energiegewinnung aus Fettsäuren im oxidativen Abbau enthaltend L-Carnitin, **dadurch gekennzeichnet, dass** L-Carnitin und/oder dessen wasserlösliche Salze bzw. C2- oder C3-Ester gemeinsam mit mit essentiellen Fettsäuren veresterten Phospholipiden, insbesondere in Form von Phosphatidylcholin (Lecithin) und/oder Phosphatidylglycerol, sowie Polyphenole als Antioxidantien enthalten sind.

2. Oral verabreichbare Zusammensetzung nach Anspruch 1, **dadurch gekennzeichnet, dass** Resveratrol als Polyphenol eingesetzt ist.

3. Oral verabreichbare Zusammensetzung nach Anspruch 1 oder 2, **dadurch gekennzeichnet, dass** Traubenkernextrakt enthalten ist.

4. Oral verabreichbare Zusammensetzung nach Anspruch 1, 2 oder 3, **dadurch gekennzeichnet, dass** zusätzlich Isoflavonoide als Antioxidantien enthalten sind.

5. Oral verabreichbare Zusammensetzung nach einem der Ansprüche 1 bis 4, **dadurch gekennzeichnet, dass** als mit essentiellen Fettsäuren veresterte Phospholipide Phosphatidylcholin (Lecithin) oder Phosphatidylglycerol, insbesondere jene Spezies, die mit Linol-, Linolensäure, Eicosapentaensäure und/oder Docosahexaensäure verestert sind, eingesetzt sind.

6. Oral verabreichbare Zusammensetzung nach einem der Ansprüche 1 bis 5, **dadurch gekennzeichnet, dass** die Zusammensetzung zusätzlich Antioxidantien aus der Gruppe der Flavonoide, Isoflavonoide, insbesondere Genistein und/oder Daidzein, Catechine, β-Carotinoide, Biotin, Vitamine C und Vitamine E enthält.

7. Oral verabreichbare Zusammensetzung nach einem der Ansprüche 1 bis 6, **dadurch gekennzeichnet, dass** als Catechin E-pigallocatechingallat (EGCG) eingesetzt ist.
